# EUROPEAN PATENT APPLICATION

(11) **EP 4 527 914 A1**
(43) Date of publication of application: **26.03.2025**
(21) Application number: 23807535.2
(22) Date of filing: 11.05.2023
(51) Int. Cl.: C12M 1/00, C12N 15/87

(54) **ELECTROPORATION DEVICE AND ELECTROPORATION METHOD**

(30) Priority: 16.05.2022 JP 2022080015
(71) Applicant: FUJIFILM Corporation, Tokyo 106-8620 (JP)
(72) Inventor: KOBAYASHI, Yuka, Ashigarakami-gun, Kanagawa 258-8577 (JP); YAKUSHIJI, Takashi, Ashigarakami-gun, Kanagawa 258-8577 (JP)
(74) Representative: Hoffmann Eitle
(86) International application number: PCT/JP2023/017697
(87) International publication number: WO 2023/223931

(57) **Abstract**

Provided are an electroporation device and an electroporation method, in which electric field concentration is suppressed. The electroporation device of the present invention is an electroporation device which introduces a bioactive substance into a biologically derived material in a suspension containing the biologically derived material and the bioactive substance. The electroporation device includes an electrode pair in which electrodes having electrode surfaces are arranged with the electrode surfaces facing each other, a power supply part which applies a voltage to the electrode pair, and a shielding part which has a higher volume resistivity than a volume resistivity of the suspension. At least a part of the shielding part is disposed in a flow passage between the facing electrode surfaces, and is disposed at a region where a distance from end part planes which are obtained by connecting outer edges of the facing electrode surfaces in a first direction orthogonal to both the facing electrode surfaces is within 2 mm in a second direction orthogonal to the first direction and parallel to the electrode surfaces.

## Description

### BACKGROUND OF THE INVENTION

### 1. Field of the Invention

The present invention relates to an electroporation device and an electroporation method, in which electrode surfaces of electrodes are arranged to face each other, and particularly relates to an electroporation device and an electroporation method, in which a shielding part that shields a current generated in a case where a voltage is applied to the electrode is provided.

### 2. Description of the Related Art

An electric perforating method is a method of introducing a substance into cells by perforating holes in a cell membrane with an electric pulse, and is also called electroporation. For example, fine holes can be made in the cell membrane by applying an electric field to a cell suspension using an electrode pair, and cells can be transformed by introducing deoxyribonucleic acid (DNA) into the cells.

A batch type is widely used as the electroporation for introducing a bioactive substance such as DNA, ribonucleic acid (RNA), and protein into a biologically derived material such as a cell, a cell derivative, a cell organelle, an intracellular granule, and a vesicle by an electric perforating method. In the batch type, for example, a suspension containing a biologically derived material and a bioactive substance is accommodated in a container having an inner surface with the electrode pair installed thereon, and an electric field is applied by the electrode pair. As a result, fine holes are produced in the membrane covering a surface of the biologically derived material, and permeability of the membrane is increased. In addition, the bioactive substance passes through the membrane with increased permeability by diffusion or electrophoresis, and thus the bioactive substance is introduced into the biologically derived material.

Meanwhile, flow-type electroporation has also been developed in which a suspension containing a biologically derived material and a bioactive substance is allowed to flow in a flow passage in which an electrode pair is installed (for example, JP2007-7430A). The flow-type is suitable for processing a large amount of suspension.

### SUMMARY OF THE INVENTION

In the flow electroporation chamber of JP2007-7430A, two electrodes facing each other are applied with an electric pulse so as to have a predetermined electric field intensity. In this case, the electric field is concentrated at an edge of the electrodes. For example, in a case where a distance between the electrodes is increased due to scaling up of the flow passage, or the like, the electric field concentration is also increased, which may cause heat generation or discharge. In a case where the heat generation or the discharge occurs, the cells are damaged or the electrodes are deteriorated, so that it is necessary to prevent the heat generation and the discharge from occurring. However, in order to prevent the heat generation and the discharge, it is necessary to suppress the electric field concentration, but the electric field concentration is not considered in JP2007-7430A.

An object of the present invention is to provide an electroporation device and an electroporation method, in which electric field concentration is suppressed.

The above-described object can be achieved by the following configurations.

As the invention [1], one aspect of the present invention is an electroporation device which introduces a bioactive substance into a biologically derived material in a suspension containing the biologically derived material and the bioactive substance, the electroporation device including an electrode pair in which electrodes having electrode surfaces are arranged with the electrode surfaces facing each other, a power supply part which applies a voltage to the electrode pair, and a shielding part which has a higher volume resistivity than a volume resistivity of the suspension, in which at least a part of the shielding part is disposed in a flow passage between the facing electrode surfaces, and is disposed at a region where a distance from end part planes which are obtained by connecting outer edges of the facing electrode surfaces in a first direction orthogonal to both the facing electrode surfaces is within 2 mm in a second direction orthogonal to the first direction and parallel to the electrode surfaces.

As the invention [2], the electroporation device according to the invention [1] further includes a container for accommodating the suspension, in which the electrodes having the electrode surfaces are disposed in the container with the electrode surfaces facing each other, the at least a part of the shielding part is disposed in the flow passage between the facing electrode surfaces which are disposed in the container, and is disposed at the region where the distance from the end part planes is within 2 mm in the second direction, and a voltage is applied to the facing electrodes by the power supply part in a state in which the suspension is accommodated in the container.

As the invention [3], in the electroporation device according to the invention [1] or [2], the shielding part is provided on at least one of an upstream side or a downstream side of the flow passage.

As the invention [4], in the electroporation device according to any one of the inventions [1] to [3], a plurality of the shielding parts are provided on at least one of an upstream side or a downstream side of the flow passage.

As the invention [5], in the electroporation device according to any one of the inventions [1] to [4], with respect to a longer distance between distances of each of the facing electrode surfaces and the shielding part, a length in the second direction from the end part plane of the shielding part to a tip portion which enters a spacing between the electrode surfaces is 1/16 or more.

As the invention [6], in the electroporation device according to any one of the inventions [1] to [5], with respect to a longer distance between distances of each of the facing electrode surfaces and the shielding part, a length in the second direction from the end part plane of the shielding part to a rear end part on a side opposite to the electrode surfaces is 1/2 or more.

As the invention [7], in the electroporation device according to the invention [4], in the plurality of the shielding parts, with respect to a longer distance between distances of each of the facing electrode surfaces and the shielding part and a distance between adjacent shielding parts in the first direction, a length in the second direction from the end part plane of the shielding part to a tip portion which enters a spacing between the electrode surfaces is 1/16 or more.

As the invention [8], in the electroporation device according to the invention [4], in the plurality of the shielding parts, with respect to a longer distance between distances of each of the facing electrode surfaces and the shielding part and a distance between adjacent shielding parts in the first direction, a length in the second direction from the end part plane of the shielding part to a rear end part on a side opposite to the electrode surfaces is 1/2 or more.

As the invention [9], in the electroporation device according to any one of the inventions [1] to [8], the shielding part is provided at at least two locations.

As the invention [10], in the electroporation device according to any one of the inventions [1] to [9], a width of the shielding part in a direction which is orthogonal to a direction from the upstream side to the downstream side of the flow passage and is parallel to the electrode surfaces is 90% or more and 100% or less with respect to a width of the flow passage.

As the invention [11], in the electroporation device according to any one of the inventions [1] to [9], the shielding part is in a sheet shape.

As the invention [12], in the electroporation device according to any one of the inventions [1] to [9], the shielding part is disposed in parallel to the electrode surfaces.

As the invention [13], in the electroporation device according to any one of the inventions [1] to [12], the electrode pair is disposed with the electrode surfaces in parallel to each other, and the shielding part is disposed at a position in which, in the facing electrode surfaces, a ratio between a distance from one electrode surface and a distance from the other electrode surface facing the one electrode surface is 3:7 to 7:3 in the first direction orthogonal to both the facing electrode surfaces. The above-described ratio is more preferably 4:6 to 6:4 and still more preferably 4.8:5.2 to 5.2:4.8.

As the invention [14], in the electroporation device according to any one of the inventions [1] to [13], the electrode pair is disposed to face each other with the electrode surfaces in parallel to each other, and the outer edges of the electrode surfaces match each other in the second direction that is orthogonal to the first direction which is a direction orthogonal to both the facing electrode surfaces, and that is parallel to the electrode surfaces. Here, the "match each other" means that the two pieces do not have to be exactly the same, but are designed to match.

As the invention [15], in the electroporation device according to any one of the inventions [1] to [14], the electrode pair is disposed with the electrode surfaces in parallel to each other, and the shielding part is disposed at a position of 0.01 mm or more and 3 mm or less from one electrode surface in the first direction orthogonal to both the facing electrode surfaces. The position is more preferably 0.5 mm or more and 2.5 m or less, and particularly preferably 1 mm or more and 2.3 mm or less from one electrode surface.

As the invention [16], in the electroporation device according to any one of the inventions [1] to [15], a thickness of the shielding part is 0.5 mm or less.

As the invention [17], in the electroporation device according to any one of the inventions [1] to [16], an elastic modulus of the shielding part is 0.1 GPa or more.

As the invention [18], in the electroporation device according to any one of the inventions [1] to [17], the volume resistivity of the shielding part is 1 × 10¹⁰ Ω·cm or more.

The invention [19] is an electroporation method including performing electroporation using the electroporation device according to any one of the inventions [1] to [18].

According to the present invention, it is possible to provide an electroporation device and an electroporation method, in which electric field concentration is suppressed.

### BRIEF DESCRIPTION OF THE DRAWINGS

Fig. 1 is a schematic plan view showing a first example of an electroporation device according to an embodiment of the present invention.
Fig. 2 is a schematic cross-sectional view showing the first example of the electroporation device according to the embodiment of the present invention.
Fig. 3 is a schematic cross-sectional view showing a second example of the electroporation device according to the embodiment of the present invention.
Fig. 4 is a schematic cross-sectional view showing a third example of the electroporation device according to the embodiment of the present invention.
Fig. 5 is a schematic view showing a calculation model of the electroporation device.

### DESCRIPTION OF THE PREFERRED EMBODIMENTS

Hereinafter, the electroporation device and the electroporation method according to the embodiment of the present invention will be described in detail based on suitable embodiments shown in the accompanying drawings.

The drawings described below are exemplary for explaining the present invention, and the present invention is not limited to the drawings illustrated below.

In the following, "to" indicating the numerical range includes numerical values described on both sides. For example, in a case where ε is a numerical value α to a numerical value β, the range of ε is a range including the numerical value α and the numerical value β, and it is expressed as α ≤ ε ≤ β in mathematical symbols.

Unless otherwise specified, angles such as "parallel" and "orthogonal" include an error range generally allowed in the relevant technical field.

### (Electroporation device)

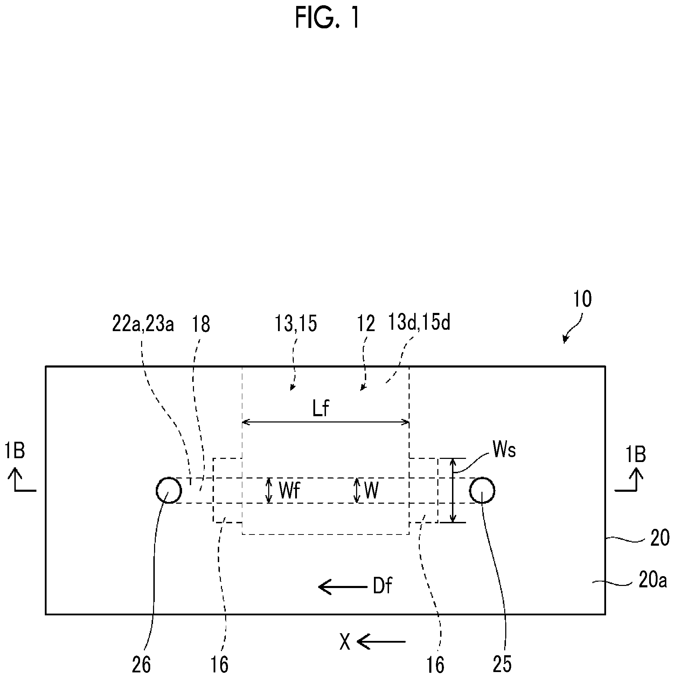

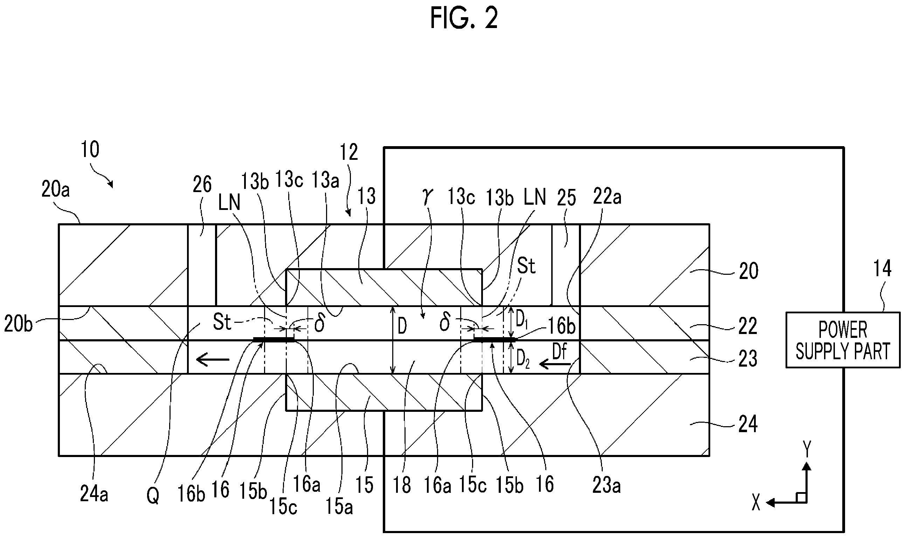

Fig. 1 is a schematic plan view showing a first example of the electroporation device according to the embodiment of the present invention. Fig. 2 is a schematic cross-sectional view showing the first example of the electroporation device according to the embodiment of the present invention.

An electroporation device 10 shown in Figs. 1 and 2 is a device which introduces a bioactive substance into a biologically derived material in a suspension Q containing the biologically derived material and the bioactive substance. The electroporation device 10 is a flow-type device, and electroporation is performed in a state in which the suspension Q flows.

The electroporation device 10 includes an electrode pair 12 in which electrodes having electrode surfaces 13a and 15a are arranged with the electrode surfaces 13a and 15a facing each other, and a shielding part 16 which has a higher volume resistivity than a volume resistivity of the suspension. At least a part of the shielding part 16 is disposed in a flow passage 18 between the facing electrode surface 13a and electrode surface 15a, and is disposed at a region St where a distance from end part planes LN which are obtained by connecting outer edges 13b and 15b of the facing electrode surfaces 13a and 15a in a first direction Y orthogonal to both the facing electrode surface 13a and electrode surface 15a is within 2 mm in a second direction X orthogonal to the first direction Y and parallel to the electrode surfaces 13a and 15a. In this case, for example, a distance D between the electrodes is 4 mm.

The shielding part 16 cut a current, particularly a current generated on the outer edges 13b and 15b of the electrode surfaces 13a and 15a, which is generated in a case where a voltage is applied to the facing electrodes by a power supply part 14. The shielding part 16 suppresses electric field concentration on a first electrode 13 and a second electrode 15, particularly at a corner portion 13c of the first electrode 13 and a corner portion 15c of the second electrode 15. In a case of performing electroporation, the electric field concentration at the corner portion 13c of the first electrode 13 and the corner portion 15c of the second electrode 15 is suppressed by the shielding part 16 in a state in which the suspension Q is present in a spacing γ between the electrode surface 13a and the electrode surface 15a. Here, a direction parallel to the electrode surfaces 13a and 15a is the second direction X described later.

More specifically, as shown in Figs. 1 and 2, the electroporation device 10 includes the electrode pair 12 including the first electrode 13 and the second electrode 15. The first electrode 13 has the electrode surface 13a, and the electrode surface 13a is a plane. The second electrode 15 has the electrode surface 15a, and the electrode surface 15a is a plane. The electrode surface 13a of the first electrode 13 and the electrode surface 15a of the second electrode 15 face each other, and are arranged in parallel to each other.

In the electroporation device 10, as shown in Fig. 2, a first electrode receiving member 20, a first flow passage plate 22, a second flow passage plate 23, and a second electrode receiving member 24 are laminated in this order. Opening portions 22a and 23a extending in one direction are formed in the first flow passage plate 22 and the second flow passage plate 23 as shown in Fig. 1 in portions corresponding to the flow passage 18, and the opening portions 22a and 23a constitute the flow passage 18 by the first flow passage plate 22 and the second flow passage plate 23 laminated as shown in Fig. 2. In addition, a back surface 20b of the first electrode receiving member 20 and a front surface 24a of the second electrode receiving member 24 constitute an inner surface of the flow passage 18.

The first electrode receiving member 20 is provided with an inflow port 25 and an outflow port 26, which communicate with the opening portion 22a of the first flow passage plate 22 and the opening portion 23a of the second flow passage plate 23.

The shielding part 16 is disposed in the flow passage 18 such that the shielding part 16 is sandwiched between the first flow passage plate 22 and the second flow passage plate 23 to be fixed in the flow passage 18. A fixing method of the shielding part 16 is not limited to being sandwiched between the first flow passage plate 22 and the second flow passage plate 23. As the fixing method of the shielding part 16, the shielding part 16 and the first flow passage plate 22 or the second flow passage plate 23 may be integrated with each other to fix the shielding part 16.

The first electrode 13 is provided on the first electrode receiving member 20 such that the electrode surface 13a faces the inner surface of the flow passage 18. For example, the first electrode 13 is fitted into the first electrode receiving member 20. The first electrode receiving member 20 is a holding member for the first electrode 13. The electrode surface 13a of the first electrode 13 constitutes the inner surface of the flow passage 18.

In addition, the second electrode 15 is provided on the second electrode receiving member 24 such that the electrode surface 15a faces the inner surface of the flow passage 18. For example, the second electrode 15 is fitted into the second electrode receiving member 24. The second electrode receiving member 24 is a holding member for the second electrode 15. The electrode surface 15a of the second electrode 15 constitutes the inner surface of the flow passage 18.

In this way, the electrode surface 13a of the first electrode 13 and the electrode surface 15a of the second electrode 15 face each other with the flow passage 18 interposed therebetween, and the electrode surfaces 13a and 15a are arranged in parallel to each other.

In addition, the first electrode 13 and the second electrode 15 of the electrode pair 12 are electrically connected to the power supply part 14. A voltage is applied to the first electrode 13 and the second electrode 15 facing each other by the power supply part 14.

The first electrode 13 includes a lead-out part 13d (see Fig. 1) which is connected integrally with the above-described electrode surface 13a. In addition, the second electrode 15 also includes a lead-out part 15d (see Fig. 1) which is connected integrally with the above-described electrode surface 15a.

The first electrode receiving member 20 is provided with a through-hole (not shown) reaching the lead-out part 13d of the first electrode 13. The second electrode receiving member 24 is provided with a through-hole (not shown) reaching the lead-out part 15d of the second electrode 15. The power supply part 14, and the first electrode 13 and the second electrode 15 are electrically connected to each other through the above-described through-holes.

In the electroporation device 10, the electroporation is performed in a state in which the flow passage 18 is allowed to flow the suspension Q.

In a case of using the electroporation device 10, a voltage is applied to the first electrode 13 and the second electrode 15 facing each other by the power supply part 14, and the suspension Q is injected from the inflow port 25, flows along the flow passage 18 in a direction Df shown in Fig. 1, and is discharged from the outflow port 26. The direction Df shown in Fig. 1 is also a direction from an upstream side to a downstream side of the flow passage 18.

A tube for the suspension may be connected to the inflow port 25 and the outflow port 26. For example, a pump (not shown) is used for the injection of the suspension Q from the inflow port 25 and the discharge of the suspension Q from the outflow port 26.

A configuration in which the inflow port 25 and the outflow port 26 are provided in the second electrode receiving member 24 instead of the first electrode receiving member 20 may be adopted, or a configuration in which the first electrode receiving member 20 has one of the inflow port 25 and the outflow port 26 and the second electrode receiving member 24 has the other of the inflow port 25 and the outflow port 26 may be adopted.

It is preferable that each of a spacing between the first electrode receiving member 20 and the first flow passage plate 22, a spacing between the first flow passage plate 22 and the second flow passage plate 23, and a spacing between the second electrode receiving member 24 and the second flow passage plate 23 is provided with a sealing member (not shown) for preventing liquid leakage of the suspension Q. The sealing member is, for example, an O-ring.

In the electrode pair 12, for example, as shown in Fig. 1, a width of the electrode surface 13a of the first electrode 13 in the flow passage 18 and a width of the electrode surface 15a of the second electrode 15 in the flow passage 18 are the same, and are both an electrode width Wf.

The width of the first electrode 13 and the second electrode 15 is not the actual width of the electrodes, but is a width of portion of the electrodes facing the flow passage 18 and corresponds to a width W (see Fig. 1) of the flow passage 18.

In addition, in the electrode pair 12, a length of the electrode surface 13a of the first electrode 13 and a length of the electrode surface 15a of the second electrode 15 are the same, and both are an electrode length Lf. In this way, the first electrode 13 and the second electrode 15 of the electrode pair 12 have the same shape as viewed from a front surface 20a side of the first electrode receiving member 20 shown in Fig. 1, for example.

In addition, for example, a direction orthogonal to both the facing electrode surfaces 13a and 15a of the first electrode 13 and the second electrode 15 is defined as the first direction Y, and the outer edges 13b and 15b of the electrode surfaces 13a and 15a are arranged to be matched with each other in the second direction X orthogonal to the first direction Y and parallel to the electrode surfaces 13a and 15a. The "match" means that the two pieces do not have to be exactly the same as described above, but are designed to match. In addition, regarding the matching of the outer edges of the electrode surfaces, in a case where the one electrode surface and the other electrode surface overlap each other by 90% or more of the electrode length Lf, the outer edges of the electrode surfaces are matched as a deviation of a relative position of the other electrode surface with respect to the one electrode surface in the second direction X.

As shown in Fig. 1, the width (electrode width Wf) of the electrode surface 13a of the first electrode 13 of the electrode pair 12 and the width (electrode width Wf) of the electrode surface 15a of the second electrode 15 are the same as the width W of the flow passage 18. An electric field is applied to the suspension Q from a portion of the electrode surface 13a of the first electrode 13 and the electrode surface 15a of the second electrode 15, facing the flow passage 18.

Here, in a case where a voltage is applied to the facing electrodes by the power supply part 14 in a state in which the suspension Q is present at the spacing γ between the electrode surface 13a and the electrode surface 15a, lines of electric force (not shown) are generated, but the lines of electric force enter an equipotential plane perpendicularly. Therefore, it is known that the electric field is concentrated at four corners in the parallel flat plate. The electrode surface 13a of the first electrode 13 and the electrode surface 15a of the second electrode 15 are arranged in parallel to each other, and the electric field is concentrated at the corner portion 13c of the first electrode 13 and the corner portion 15c of the second electrode 15.

The shielding part 16 cuts a current generated in a case where the voltage is applied to the facing electrodes as described above, and the shielding part 16 suppresses the electric field concentration of the first electrode 13 and the second electrode 15.

The shielding part 16 has a volume resistivity higher than that of the suspension Q. That is, in a case where a volume resistivity of the shielding part 16 is denoted by ρ₁ and a volume resistivity of the suspension Q is denoted by ρ₂, ρ₁ > ρ₂.

The volume resistivity ρ₁ of the shielding part 16 is calculated by ρ₁ (Ω·m) = R₁ × (A₁/t₁) from a plane area A₁ and a thickness t₁ of the shielding part 16 after measuring an electrical resistance R₁.

In a case where a commercially available product is used as the shielding part 16, the volume resistivity of the shielding part 16 is a catalog value of the commercially available product.

The volume resistivity of the suspension Q is obtained by measuring the voltage and the current of the suspension Q using an oscilloscope to obtain an electric resistance R₂ of the suspension Q. Next, the volume resistivity ρ₂ (Ω·m) is calculated by ρ₂ (Ω·m) = R₂ × (A₂/t₂) from an area A₂ of a plane of the flow passage of the suspension Q parallel to the electrode surface, and a distance t₂ between electrodes of the electrode surfaces.

In a case where a commercially available product is used as the suspension Q, the volume resistivity of the suspension Q is a catalog value of the commercially available product.

In a case where a voltage is applied between the first electrode 13 and the second electrode 15 by the power supply part 14, an electric field is generated between the first electrode 13 and the second electrode 15, and the electric field is concentrated at the corner portion 13c of the first electrode 13 and the corner portion 15c of the second electrode 15, so that the corner portion 13c of the first electrode 13 and the corner portion 15c of the second electrode 15 are in a state in which a current density is high. In this case, a current also flows between the first electrode 13 and the second electrode 15.

However, in a case where the voltage is applied, the current generated between the corner portion 13c of the first electrode 13 and the corner portion 15c of the second electrode 15 is cut by the shielding part 16, and the electric field concentration of the corner portion 13c of the first electrode 13 and the electric field concentration of the corner portion 15c of the second electrode 15 are relaxed, so that the electric field concentration is suppressed. That is, the current density of the corner portion 13c of the first electrode 13 and the current density of the corner portion 15c of the second electrode 15 are reduced. From the above, even in a case where the distance between the electrodes is increased due to scale-up of the flow passage, or the like, the increase in the electric field concentration can be suppressed, the occurrence of discharge can be suppressed, the damage of the cells can be suppressed, and the deterioration of the electrodes can be suppressed. The degree of suppression of the electric field concentration varies depending on the position of the shielding part 16, as described later, and in the shielding part 16, the electric field concentration between the corner portion 13c of the first electrode 13 and the corner portion 15c of the second electrode 15 can be further suppressed by being located at the spacing γ between the electrode surface 13a and the electrode surface 15a.

Although the electric field is concentrated at the end part of the shielding part 16, different from the electrode in which a gas is generated on the surface by electrolysis of the suspension Q, no gas is generated on the surface of the shielding part 16, so that the discharge does not occur.

For example, the shielding part 16 is in a sheet shape. It is preferable that the shielding part 16 is in a sheet shape, because it is possible to suppress disturbance of the flow of the suspension Q flowing through the flow passage 18.

In addition, it is preferable that the shielding part 16 is disposed in parallel with the electrode surface 13a and the electrode surface 15a, because it is possible to suppress disturbance of the flow of the suspension Q flowing through the flow passage 18. Here, as the parallel disposition of the electrode surfaces 13a and 15a and the shielding part 16, an inclination of the shielding part 16 up to ±5° with respect to the electrode surface 13a and the electrode surface 15a is set to an allowable range. In addition, in a case where the electrode surfaces 13a and 15a are arranged in parallel to each other, an inclination of one electrode surface up to ±5° with respect to the other electrode surface is set to an allowable range.

In addition, the shielding part 16 is disposed at a position in which, in the facing electrode surfaces, a ratio between a distance from one electrode surface and a distance from the other electrode surface facing the one electrode surface is preferably 3:7 to 7:3, more preferably 4:6 to 6:4, and still more preferably 4.8:5.2 to 5.2:4.8 in the first direction Y orthogonal to both the facing electrode surfaces.

More specifically, the distance between the electrodes in the spacing γ between the electrode surface 13a and the electrode surface 15a in the first direction Y, as shown in Fig. 2, is denoted by D. In addition, in the facing electrode surfaces, a distance between the electrode surface 13a corresponding to one electrode surface and the shielding part 16 is denoted by D₁; and a distance between the electrode surface 15a corresponding to the other facing electrode surface and the shielding part 16 is denoted by D₂. The shielding part 16 is disposed at a position in which a ratio between the distance D₁ and the distance D₂, that is, D₁:D₂ is preferably 3:7 to 7:3, more preferably 4:6 to 6:4, and still more preferably 4.8:5.2 to 5.2:4.8.

In addition, it is preferable that the shielding part 16 is disposed at a position of D/2 from the electrode surface 13a and at a position of D/2 from the electrode surface 15a. An intermediate of the spacing γ between the electrode surface 13a and the electrode surface 15a is set to an allowable range of 10% of the distance as an error. By disposing the shielding part 16 at the intermediate of the spacing γ between the electrode surface 13a and the electrode surface 15a, the electric field concentration of the first electrode 13 and the electric field concentration of the second electrode 15 can be suppressed to the same extent.

The shielding part 16 may be disposed at a position of 0.01 or more and 3 mm or less from one of the electrode surfaces 13a and 15a, and in this case, it is preferable that the shielding part 16 is disposed closer to a negative pole side of the electrodes. The shielding part 16 is more preferably disposed at a position of 0.5 mm or more and 2.5 m or less from one of the electrode surfaces 13a and 15a, and particularly preferably disposed at a position of 1 mm or more and 2.3 mm or less from one of the electrode surfaces 13a and 15a. As a result, the effect of suppressing the electric field concentration can be enhanced.

As described above, the shielding part 16 is disposed at the region St in the spacing γ between the facing electrode surface 13a and electrode surface 15a, where a distance from end part planes LN which are obtained by connecting the outer edges 13b and 15b of the facing electrode surfaces 13a and 15a in the first direction Y is within 2 mm in the second direction X. As the above-described region St, based on the end part planes LN obtained by connecting the outer edges 13b and 15b of the electrode surfaces 13a and 15a in the first direction Y, a region where a distance δ on a side of the spacing γ between the electrode surface 13a and the electrode surface 15a is up to 2 mm and a distance δ on a side opposite to the spacing γ between the electrode surface 13a and the electrode surface 15a is up to 2 mm is included in the above-described region St. By disposing the shielding part 16 at the above-described region St, the effect of suppressing the electric field concentration is obtained.

In a case where the shielding part 16 is disposed at the above-described region St, at least a part of the shielding part 16 may be disposed in the above-described region St.

In the above-described region St, it is preferable that at least a part of the shielding part 16 is located at the spacing γ between the electrode surface 13a and the electrode surface 15a in the second direction X with respect to the end part planes LN obtained by connecting the outer edges 13b and 15b of the electrode surfaces 13a and 15a in the first direction Y. As a result, the electric field concentration of the electrode pair 12 can be suppressed.

In Fig. 2, in the above-described region St, a tip portion 16a of the shielding part 16 is located at the spacing γ between the electrode surface 13a and the electrode surface 15a.

At least a part of the shielding part 16 is located at a portion of 2 mm on the side of the spacing γ between the electrode surface 13a and the electrode surface 15a in the second direction X, from the end part planes LN obtained by connecting the outer edges 13b and 15b of the electrode surfaces 13a and 15a in the first direction Y. That is, the position of the tip portion 16a of the shielding part 16 is a position where the distance δ between the end part plane LN and the tip portion 16a in the second direction X is 2 mm. As a result, the electric field concentration of the electrode pair 12 can be suppressed.

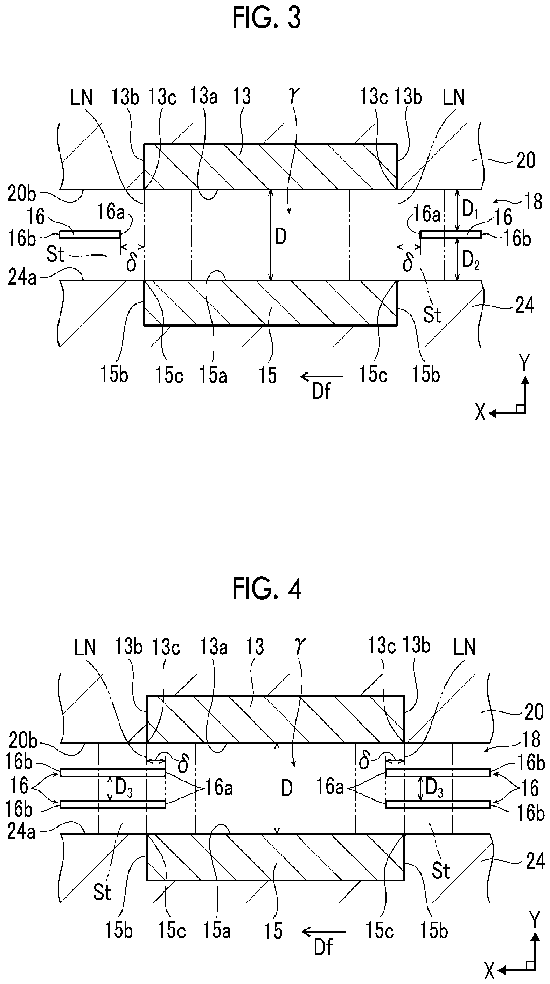

Fig. 3 is a schematic cross-sectional view showing a second example of the electroporation device according to the embodiment of the present invention. Fig. 4 is a schematic cross-sectional view showing a third example of the electroporation device according to the embodiment of the present invention. In Figs. 3 and 4, the same components as those of the electroporation device 10 shown in Figs. 1 and 2 are designated by the same reference numerals, and the detailed description thereof will be omitted. In addition, Figs. 3 and 4 show the arrangement of the first electrode 13, the second electrode 15, and the shielding part 16, and the illustration of the other configurations of the electroporation device 10 is omitted.

As shown in Fig. 3, the tip portion 16a of the shielding part 16 may be located on a side opposite to the spacing γ of the electrode surface 13a and the electrode surface 15a with respect to the end part planes LN obtained by connecting the outer edges 13b and 15b of the electrode surfaces 13a and 15a in the first direction Y, as long as the tip portion 16a is located in the above-described region St. That is, the shielding part 16 may be provided outside the spacing γ between the electrode surface 13a and the electrode surface 15a. Even in the configuration in which the shielding part 16 is disposed as described above, the effect of suppressing the electric field concentration can be obtained. The position of the tip portion 16a of the shielding part 16 shown in Fig. 3 indicates that, for example, in a case which the distance D between the electrodes is 4 mm, the distance δ between the end part plane LN and the tip portion 16a in the second direction X is 1 mm.

In addition, in a case where the distance D between the electrodes is long and the electric field concentration is large, a plurality of the shielding parts 16 may be provided. As a result, the electric field concentration can be suppressed even in a case where the electric field concentration is large. More specifically, as shown in Fig. 4, two shielding parts 16 may be provided on both the upstream side and the downstream side of the flow passage 18. In this case, the number of the shielding parts 16 is 4 in total. In addition, for example, the two shielding parts 16 described above have the same size, and are laminated and disposed in the first direction Y. In this case, for example, the two shielding parts 16 are arranged at equal intervals with respect to the distance D between the electrodes, but the arrangement at equal intervals is not limited thereto and the two shielding parts 16 may be arranged close to the negative pole side of the electrodes.

Even in a case where the two shielding parts 16 are arranged, the position of the tip portion 16a is not particularly limited as long as it is in the above-described region St, and as shown in Fig. 2, the tip portion 16a may be located at the spacing γ between the electrode surface 13a and the electrode surface 15a, may coincide with the above-described end part plane LN, or as shown in Fig. 3, the tip portion 16a may be located on a side opposite to the spacing γ between the electrode surfaces 13a and 15a with respect to the end part plane LN.

In Figs. 1, 3, and 4, the configuration in which the shielding part 16 is provided on both the upstream side and the downstream side of the flow passage 18 has been adopted, but in a case where the shielding part 16 is provided on both the upstream side and the downstream side, the electric field concentration of the corner portions 13c and 15c on both sides of the first electrode 13 and the second electrode 15 can be suppressed, so that it is preferable to provide the shielding part 16 in at least two locations.

However, the present invention is not limited thereto, and it is sufficient that the shielding part 16 is provided on at least one of the upstream side or the downstream side of the flow passage 18. Furthermore, among the upstream side and the downstream side, the shielding part 16 may be configured to be provided only on the downstream side of the flow passage 18. In a case where a voltage is applied from the power supply part 14, the first electrode 13 and the second electrode 15 generate heat due to Joule heat caused by electrical resistance, and the heat is transferred to the suspension Q. As a result, a temperature of the suspension Q is higher on the downstream side of the flow passage 18 than on the upstream side. Therefore, in a case where the electric field concentration is suppressed only on the downstream side of the flow passage 18, the Joule heat on the downstream side of the flow passage 18 can be reduced, which is effective for the electroporation device 10.

The upstream side of the flow passage 18 refers to a side of the inflow port 25 shown in Figs. 1 and 2. The downstream side of the flow passage 8 refers to a side of the outflow port 26 shown in Figs. 1 and 2.

In the electroporation device 10, a method of fixing the first electrode receiving member 20, the first flow passage plate 22, the second flow passage plate 23, and the second electrode receiving member 24 shown in Fig. 2 is not particularly limited; and examples thereof include a method of forming a through-hole for assembling each member, and then fixing the through-hole with a screw, and a method of fixing the through-hole with an adhesive.

The electroporation device 10 may have a configuration including a cooling mechanism which cools each electrode of the electrode pair.

For example, a cooling flow passage (not shown) is provided on a back surface portion side which is on a side opposite to the electrode surface 13a of the first electrode 13. Similarly, a cooling flow passage (not shown) is provided on a back surface portion side which is on a side opposite to the electrode surface 15a of the second electrode 15. A tube (not shown) for a refrigerant may be attached to each of the inlet and the outlet of the cooling flow passage (not shown). A pump (not shown) is connected to the tube for a refrigerant, and the refrigerant is circulated. For the refrigerant, for example, water is used. The cooling mechanism is configured by the cooling flow passage, the tube for a refrigerant, the pump, and the refrigerant. The cooling mechanism is not limited thereto.

In a case of using the electroporation device 10, the electrode pair generates heat. As a result, the damage to the biologically derived material or the bioactive substance in the suspension may proceed, which may lead to a decrease in survival rate or introduction efficiency of the biologically derived material such as a cell. Since the cooling mechanism is provided, the heat generation of the electrode pair can be suppressed, so that the decrease in survival rate or introduction efficiency of the biologically derived material such as a cell can be suppressed. The introduction efficiency is a proportion of the biologically derived material introduced into the bioactive substance subjected to the electroporation.

### [Electrode length Lf]

The electrode length Lf is a length in the second direction X, and is not particularly limited, but from the viewpoint of introduction efficiency and the like, it is preferably 1 to 100 mm and more preferably 10 to 50 mm.

### [Electrode width Wf]

The electrode width Wf is the width of the first electrode 13 and the width of the second electrode 15, and is not particularly limited, but it is preferably 1 to 100 mm, more preferably 2 to 30 mm, and particularly preferably 5 to 25 mm.

From the reason that the introduction efficiency is excellent, the electrode width Wf is preferably 5 to 25 mm. The electrode width Wf is a length in a direction orthogonal to both the first direction Y and the second direction X.

### [Distance D between electrodes]

The distance D between the electrodes of the electrode pair 12 (a distance between the electrode surface 13a of the first electrode 13 and the electrode surface 15a of the second electrode 15 in the first direction Y) is preferably 1 to 10 mm and more preferably 2 to 8 mm.

In a case where the distance D between the electrodes is too small, a ratio represented by electrode surface area/electrode spatial volume is increased, and the activity and the influence of oxygen species on the suspension are increased, which may reduce the survival rate of the biologically derived material such as a cell. In addition, from the viewpoint of treatment flow rate of the suspension Q, the distance D between the electrodes is more preferably 3 to 6 mm.

In addition, in a case where the distance D between the electrodes is 1 to 3 mm, the generation of heat and the discharge are suppressed, and the survival rate or the introduction efficiency of the biologically derived material such as a cell, due to the heat generation or the discharge, is also suppressed, which is more preferable.

The distance D between the electrodes corresponds to the total thickness of the first flow passage plate 22 and the second flow passage plate 23. The above-described total thickness is a length in the first direction Y, in which the first flow passage plate 22 and the second flow passage plate 23 are combined. The distance D between the electrodes is also referred to as an inter-electrode gap or a gap.

### <Suspension>

The suspension used in the electroporation device contains a biologically derived material and a bioactive substance.

### (Biologically derived material)

The biologically derived material is not particularly limited, and specific examples thereof include cells, cell organelles, intracellular granules and vesicles, and bacteria. Among these, the biologically derived material is preferably a cell, more preferably an animal cell, still more preferably a mammalian cell, and most preferably a human-derived cell due to the reason that the effects and the like of the present invention are more excellent. Specific examples of the cell include human T-cell, human embryonic kidney (HEK) 293 cells, A549, SF9, EB66, Daudi, Hela, Vero, MDCK, baby hamster kidney (BHK), Chinese hamster ovary (CHO), NS0, SP2/0, and hybridoma. In view of medicinal chemical manufacturing, gene introduction into HEK 293 cells or CHO cells is most commonly used.

### (Bioactive substance)

The bioactive substance is a substance such as DNA, RNA, and protein, which exerts some action on a biologically derived material by being introduced into the biologically derived material. The bioactive substance is, for example, plasmid, linear DNA, messenger RNA (mRNA), or protein, preferably plasmid, mRNA, or linear DNA, and particularly preferably plasmid.

### <Voltage of electroporation>

In general, the voltage is set according to the gap between the electrodes (distance D between the electrodes) such that the electric field applied to the suspension between the electrodes reaches a desired electric field strength.

In a case where electric resistances of the electrodes in a thickness direction and an in-plane direction are sufficiently small, the value of the strength of the electric field applied to the suspension between the electrodes is obtained by dividing the voltage applied from an external power supply by the distance D between the electrodes, and a uniform electric field is formed regardless of the position of the liquid contact surface.

On the other hand, in a case where electric resistance values of the electrodes in the thickness direction and the in-plane direction are high, a part of the voltage applied from the external power supply is consumed in the electrode portion, and thus a decrease in effective strength of the electric field applied to the suspension or a distribution of the electric field strength in the liquid contact surface occurs, which causes a decrease in electroporation efficiency.

An optimal value of the electric field of the electroporation varies depending on the type of the biologically derived material, and the like, but is usually approximately 1,000 to 2,000 V/cm.

The voltage of the electroporation is preferably a pulse voltage. In addition, a bipolar pulse (alternating between a positive pulse and a negative pulse) may be provided in order to make electrode reaction (gas generation by electrolysis, electrode degradation, and the like) uniform.

### <Pulse width>

An optimum value of a pulse width varies depending on the type of the biologically derived material, and the like, but is usually 0.1 to 100 ms (milliseconds), preferably approximately 1 to 10 ms.

### <Pulse period (pulse interval)>

The pulse period is preferably synchronized with (an integral multiple of) a time (determined by the flow rate and the flow passage cross-sectional area (electrode width Wf × distance D between electrodes)) in which the biologically derived material passes through the electrode (electrode length L). For example, a pulse voltage is applied an average of 1 to 5 times, and preferably once while the biologically derived material passes through the electrodes (electrode length L).

The voltage of the electroporation, the pulse width, and the pulse period described above are set in the power supply part 14. The voltage of the electroporation also includes the pulse voltage and the bipolar pulse described above. The power supply part 14 performs the electroporation based on a set voltage of the electroporation, a set pulse width, and a set pulse period.

### (Electroporation method)

Next, an electroporation method using the electroporation device 10 shown in Fig. 1 will be described.

In the electroporation device 10, a pulse voltage is applied to the first electrode 13 and the second electrode 15 by the power supply part 14, for example. As a result, an electric field is formed between the first electrode 13 and the second electrode 15, but the electric field concentration between the first electrode 13 and the second electrode 15 is suppressed by the shielding part 16.

In a case where the suspension Q is injected from the inflow port 25, the injected suspension Q flows through the flow passage 18 in the direction Df shown in Fig. 1 and is discharged from the outflow port 26. Since the power supply part 14 applies the electric field in a case where the suspension Q passes through the flow passage 18, the surface of the biologically derived material in the suspension is covered with the film in which the minute pores are opened, and permeability of the film is increased. As a result, the bioactive substance in the suspension is introduced into the biologically derived material by diffusion or electrophoresis. In this way, the electroporation is carried out in a state in which the suspension Q flows in the flow-type electroporation device 10.

In the electroporation method, since the electric field concentration between the first electrode 13 and the second electrode 15 is suppressed by the shielding part 16, the generation of discharge is suppressed, the damage to the biologically derived material and the bioactive substance in the suspension is also suppressed, and the deterioration of the electrodes is further suppressed.

The flow-type electroporation device 10 shown in Figs. 1 and 2 is not limited, and for example, an electroporation device used for batch-type electroporation may be used.

In a case of the batch type, the above-described biologically derived material is introduced into the above-described bioactive substance by applying an electric field to the suspension Q containing the biologically derived material and the bioactive substance using an electrode pair.

An electroporation device used for the batch-type electroporation includes a container for accommodating the suspension. The electrodes having the electrode surfaces are disposed in the container with the electrode surfaces facing each other. Specifically, the electrode surface 13a (see Fig. 2) of the first electrode 13 (see Fig. 2) described above and the electrode surface 15a (see Fig. 2) of the second electrode 15 (see Fig. 2) are disposed in the container to face each other. The shielding part 16 (see Fig. 2) is disposed in the same manner as the electroporation device 10 (see Fig. 2) described above. In addition, at least a part of the shielding part 16 is disposed in the flow passage between the facing electrode surfaces disposed in the container, and is disposed in a region where a distance from the end part planes LN in the second direction X is 2 mm or less.

For example, in a state in which the container accommodates the suspension Q, the electroporation is carried out by applying a voltage to the facing electrodes, that is, the first electrode 13 and the second electrode 15 by the power supply part 14 (see Fig. 2). In the batch-type electroporation device, the same effect as in the electroporation device 10 described above can be obtained, and the electric field concentration can be suppressed.

The electroporation device used for the batch-type electroporation may be a single batch type or a repeated batch type, but is preferably a repeated batch type. In addition, in the batch type, since the configuration is not a configuration in which the suspension Q flows, the flow passage may not be strictly a flow passage, but a region between the facing electrode surfaces and the periphery thereof is collectively defined as the flow passage, as in the flow type.

### [Member]

Next, each member used in the electroporation device will be described.

### <Electrode pair>

The first electrode 13 and the second electrode 15 constituting the electrode pair are made of, for example, a metal material.

### (Metal material)

The metal material is not particularly limited, but from the viewpoint of thermal conductivity and the like, Mo (molybdenum), Nb (niobium), W (tungsten), or Ti (titanium) is preferable; and Mo is more preferable.

### (Thickness)

A thickness of the first electrode 13 and the second electrode 15 is not particularly limited, but is preferably 0.5 to 10 mm and more preferably 1 to 5 mm.

### <Electrode holding member>

A material constituting the first electrode receiving member 20 which is a holding member for the first electrode 13 and a material constituting the second electrode receiving member 24 which is a holding member for the second electrode 15 are not particularly limited, and a material having high workability and capable of withstanding autoclave sterilization at a temperature of 121°C for 20 minutes or more is preferable. Examples of such a material include polycarbonate and polypropylene.

### <Flow passage plate>

Materials constituting the first flow passage plate 22 and the second flow passage plate 23 are not particularly limited, and from the viewpoint of reducing adhesion of the biologically derived material such as a cell, preventing damage due to heat generation and discharge during electroporation, a fluororesin material such as Teflon (registered trademark) is preferable.

In a case where it is difficult to prevent liquid leakage with a sealing member such as an O-ring, the first flow passage plate 22 and the second flow passage plate 23 can also serve as the sealing member by using a material having flexibility and weak adhesiveness, such as polydimethylsiloxane (PDMS).

### <Shielding part>

The shielding part 16 is made of, for example, polytetrafluoroethylene (PTFE). An elastic modulus of PTFE is, for example, 0.6 GPa at a temperature of 25°C. In a case where a commercially available product of PTFE is used, the elastic modulus is a catalog value of the commercially available product.

In addition, the volume resistivity of the shielding part 16 is preferably 1 × 10¹⁰ Ω·cm or more because the effect of suppressing the electric field concentration is increased. In a case where a commercially available product is used as the shielding part 16, the volume resistivity of the shielding part 16 is a catalog value of the commercially available product.

Here, a side in the spacing γ between the electrode surface 13a and the electrode surface 15a in the second direction X with the end part plane LN as a boundary is referred to as the inside of the electrode surfaces 13a and 15a. In addition, a side opposite to the spacing γ between the electrode surface 13a and the electrode surface 15a in the second direction X with the end part plane LN as a boundary is referred to as the outside of the electrode surfaces 13a and 15a.

In a case where at least a part of the shielding part 16, for example, the tip portion 16a, enters the inside of the spacing γ (see Fig. 3) between the electrode surfaces 13a and 15a and the tip portion 16a is located on the inside, it is preferable that a length of the shielding part 16 from the end part plane LN to the tip portion 16a in the second direction X is preferably 0.5 mm or more and 5 cm or less, more preferably 1 mm or more and 1 cm or less, and particularly preferably 2 mm or more and 1 cm or less. Since the effect of suppressing the electric field concentration described above is obtained in a case where the shielding part 16 enters the inside of the spacing γ between the electrode surfaces 13a and 15a, it is preferable that the length of the shielding part 16 from the end part plane LN to the tip portion 16a in the second direction X is within the above-described range.

The length of the shielding part 16 from the end part plane LN to the tip portion 16a in the second direction X is referred to as an inner length of the shielding part 16.

In addition, a length of the shielding part 16 from the end part plane LN of the shielding part 16 to a rear end part 16b on a side opposite to the spacing γ between the electrode surfaces 13a and 15a (outside the spacing γ between the electrode surfaces 13a and 15a) in the second direction X is preferably 2 mm or more and 20 cm or less, more preferably 8 mm or more and 8 cm or less, and particularly preferably 20 mm or more and 5 cm or less.

In a case where the length from the end part plane LN of the shielding part 16 to the rear end part 16b on a side opposite to the spacing γ between the electrode surfaces 13a and 15a in the second direction X is within the above-described range, the current flowing outside the spacing γ between the electrode surfaces 13a and 15a can be shielded, which is preferable.

The length from the end part plane LN of the shielding part 16 to the rear end part 16b on a side opposite to the spacing γ between the electrode surfaces 13a and 15a (outside the spacing γ between the electrode surfaces 13a and 15a) in the second direction X is referred to as an outer length of the shielding part 16.

Among the distance D₁ between the electrode surface 13a and the shielding part 16 described above, the distance D₂ between the electrode surface 15a and the shielding part 16 described above, and a distance D₃ between adjacent shielding parts 16 in the first direction Y, the longest distance is denoted by Dy. In addition, the inner length of the shielding part 16 described above is denoted by Din. The inner length Din of the shielding part 16 with respect to the above-described longest distance Dy is preferably 1/16 (= Din/Dy) or more and 2 or less, more preferably 1/4 or more and 1 or less, and particularly preferably 1/2 or more and 3/4 or less.

In addition, the outer length of the shielding part 16 described above is denoted by Dout. The outer length Dout of the shielding part 16 with respect to the above-described longest distance Dy is preferably 1/2 (= Dout/Dy) times or more and 20 times or less, more preferably 2 times or more and 15 times or less, and particularly preferably 5 times or more and 10 times or less.

In a case where there are a plurality of the shielding parts, the distance D₃ between adjacent shielding parts 16 exists, but in a case where there is one shielding part 16, the distance D₃ between adjacent shielding parts 16 does not exist. In this case, the longest distance Dy is the distance D₁ or the distance D₂. The distance D₁ and the distance D₂ may be the same. In this case, the distance between the shielding part 16 and the electrode surface in the first direction Y is the longest distance Dy.

The length of the shielding part 16 can be measured using a caliper or a micrometer, including the inner length Din of the shielding part 16 and the outer length Dout of the shielding part 16 described above. In addition, the distance D₃ between adjacent shielding parts 16 can also be measured using a caliper or a micrometer.

A thickness of the shielding part 16 is preferably 0.5 mm or less, and more preferably 0.1 to 0.2 mm. In a case where the thickness of the shielding part 16 is 0.5 mm or less as described above, the flow of the suspension Q in the flow passage 18 is not hindered, which is preferable. The thickness of the shielding part 16 is a length of the shielding part 16 in the first direction Y. The thickness of the shielding part 16 can be measured using a micrometer.

An elastic modulus of the shielding part 16 is preferably 0.1 GPa or more. In a case where the elastic modulus is 0.1 GPa or more, when the suspension Q is allowed to flow in the flow passage 18, the flow of the suspension Q is not hindered by vibration or deformation of the shielding part 16, which is preferable. In a case where a commercially available product is used as the shielding part 16, the elastic modulus of the shielding part 16 is a catalog value of the commercially available product.

It is preferable that the width Ws (see Fig. 1) of the shielding part 16 in a direction which is orthogonal to a direction from the upstream side to the downstream side of the flow passage 18 and is parallel to the electrode surfaces is 90% or more and 100% or less with respect to the width W (see Fig. 1) of the flow passage 18. In a case where the width Ws of the shielding part 16 is within the above-described range, the suppression of the electric field concentration is sufficiently obtained, which is preferable. The direction from the upstream side to the downstream side of the flow passage 18 is the direction Df shown in Fig. 1, as described above.

The width W of the flow passage 18 and the width Ws of the shielding part 16 can be measured using a micrometer.

The present invention is basically configured as described above. The electroporation device and the electroporation method according to the embodiments of the present invention have been described in detail above, but the present invention is not limited to the above-described embodiments, and various improvements and changes can be made without departing from the spirit of the present invention.

### Examples

Hereinafter, the characteristics of the present invention will be described in detail by Examples. The materials, reagents, amounts and proportions of substances, operations, and the like described in the following examples can be appropriately modified as long as the gist of the present invention is maintained. Therefore, the scope of the present invention is not limited to Examples shown below.

In the present example, the effect of suppressing the electric field concentration by the shielding part was confirmed by performing electromagnetic field analysis using a calculation model with each of Examples 1 to 3 and Comparative Example 1.

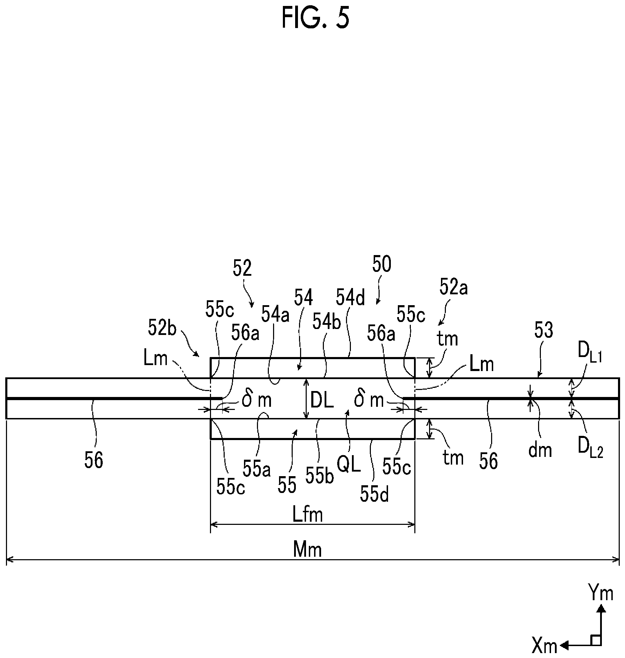

Fig. 5 is a schematic view showing a calculation model of the electroporation device. The electromagnetic field analysis was performed using a calculation model 50 shown in Fig. 5 to obtain a current density generated in a case where a voltage was applied to the electrodes of the electrode pair.

The calculation model 50 shown in Fig. 5 is a numerical analysis model capable of numerical computation using a computer; and each element of the calculation model 50 in the following description is capable of numerical computation using a computer, although the details thereof will not be described.

The calculation model 50 includes an electrode pair element 52 imitating the electrode pair 12 (see Fig. 2) and a flow passage element 53 imitating the flow passage 18 (see Fig. 2).

The flow passage element 53 is formed in a shape of a rectangle, and is formed for imitating a straight flow passage.

The electrode pair element 52 includes a first electrode element 54 imitating the first electrode 13 (see Fig. 2) and a second electrode element 55 imitating the second electrode 15 (see Fig. 2), and the first electrode element 54 and the second electrode element 55 are each rectangular.

An inflation mesh is used for the calculation model 50. The inflation mesh is created by setting to generate a mesh including five layers of laminar elements. The element size is set to 0.5 mm, the migration ratio wis set to 0.272, and the growth rate is set to 1.2. The inflation mesh is generally referred to as a layer mesh.

The first electrode element 54 and the second electrode element 55 were disposed in a direction Ym orthogonal to a longitudinal direction Xm of the flow passage element 53 with the flow passage element 53 interposed therebetween.

A shielding part element 56 was disposed in the flow passage element 53 at a position of half in the direction Ym described above, that is, at a position of DL/2 between an upstream side 52a and a downstream side 52b of the electrode pair element 52. A position of a tip portion 56a of the shielding part element 56 was adjusted with respect to an end part plane Lm connecting an end part 54c of the first electrode element 54 and an end part 55c of the second electrode element 55 to obtain the model of each of Examples. The end part plane Lm corresponds to the above-described end part plane LN (see Fig. 2). The shielding part element 56 is formed for imitating the shielding part 16 (see Fig. 2).

The total length Mm of the calculation model 50 in the longitudinal direction Xm was set to 60.0 mm. A length Lfm of the first electrode element 54 and the second electrode element 55 in the longitudinal direction Xm was set to 20.00 mm. In addition, a thickness tm of the first electrode element 54 and the second electrode element 55 in the direction Ym was set to 2.00 mm.

A distance DL between an electrode surface 54a of the first electrode element 54 and an electrode surface 55a of the second electrode element 55 was set to 4.00 mm. A thickness dm of the shielding part element 56 in the direction Ym was set to 0.10 mm, and a distance DL₁ between the shielding part element 56 and the electrode surface 54a of the first electrode element 54 and a distance DL₂ between the shielding part element 56 and the electrode surface 55a of the second electrode element 55 were set to 1.95 mm, respectively. In addition, a distance between the tip portion 56a of the shielding part element 56 and the end part plane Lm was denoted by δm. Based on the end part plane Lm, the distance δm was defined to be positive in a case where the tip portion 56a of the shielding part element 56 was on a side of the first electrode element 54, and was defined to be negative in a case where the tip portion 56a of the shielding part element 56 was on a side opposite to the first electrode element 54.

A physical property value corresponding to the suspension Q was set in the flow passage element 53. As the physical property value of the suspension Q, a physical property value of 0.9% by mass of physiological saline was set, and a volume resistivity was set to 0.625 (Ω·m).

The volume resistivity of the shielding part element 56 was set to 1.00 × 10²⁰ (Ω·m).

The volume resistivity of the first electrode element 54 and the second electrode element 55 was set to 1.61 × 10⁻⁷ (Ω·m).

The volume resistivity of the shielding part element 56 was set to be higher than the volume resistivity of the flow passage element 53.

A potential of 42 V was applied to the surface 54d of the first electrode element 54 and a potential of 0 V was applied to the surface 55d of the second electrode element 55, so that a voltage of 42 V was applied between the first electrode element 54 and the second electrode element 55. In addition, a potential condition of the boundary other than this was set to a current value = 0 A (ampere). The electromagnetic field analysis was performed under such a boundary condition. The electromagnetic field analysis was performed by the finite element method using ANSYS 2020 R1 to obtain a current density.

Hereinafter, Examples 1 to 3 and Comparative Example 1 will be described.

In Example 1, in the above-described calculation model 50, the tip portion 56a of the shielding part element 56 was disposed to advance 2 mm from the end part plane Lm between the first electrode element 54 and the second electrode element 55. That is, in Example 1, δm was set to +2 mm.

Example 2 was the same as in Example 1, except that the tip portion 56a of the shielding part element 56 was disposed to match the end part plane Lm. That is, in Example 2, δm was set to 0 mm.

Example 3 was the same as in Example 1, except that the tip portion 56a of the shielding part element 56 was disposed at a position 1 mm opposite to the end part plane Lm with respect to the first electrode element 54 and the second electrode element 55. That is, in Example 3, δm was set to -1 mm.

Comparative Example 1 was the same as in Example 1, except that the configuration in which the shielding part element 56 was not provided was adopted.

For the current density generated in a case where a voltage was applied to the electrodes of the electrode pair obtained by the electromagnetic field analysis, a ratio between the value of the current density at a center 54b of the first electrode element 54 and the maximum value of the current density at the end part 54c was defined as an electric field concentration factor (= (Maximum value of current density at end part 54c of first electrode element 54)/(Value of current density at center 54b of first electrode element 54)). The electric field concentration factor of Examples 1 to 3 and Comparative Example 1 was obtained. The results are shown in Table 1.

**[Table 1]**

| | Presence or absence of shielding part element (shielding part) | Position | Thickness (mm) | Maximum value of current density at end part of first electrode element (A/m²) | Value of current density at center of first electrode element (A/m²) | Electric field concentration factor |
|---|---|---|---|---|---|---|
| Example 1 | Y | 2 mm from inside | 0.1 | 16982.4 | 16801.5 | 1.01 |
| Example 2 | Y | 0 mm | 0.1 | 27198.4 | 16801.3 | 1.62 |
| Example 3 | Y | 1 mm from outside | 0.1 | 34706.5 | 16801.8 | 2.07 |
| Comparative Example 1 | N | - | - | 37179.7 | 16801.5 | 2.21 |

As shown in Table 1, in Examples 1 to 3, the electric field concentration factor was smaller than that in Comparative Example 1 in which the shielding part element 56 was not provided, that is, the shielding part 16 was not provided, and the electric field concentration could be suppressed.

From Examples 1 to 3, the position of the shielding part element 56, that is, the position of the shielding part 16, was such that the electric field concentration factor was smaller in a case of being inserted between the first electrode element 54 and the second electrode element 55, and the effect of suppressing the electric field concentration was excellent.

### Explanation of References

10: electroporation device
12: electrode pair
13: first electrode
13a, 15a, 54a, 55a: electrode surface
13b, 15b: outer edge
13c, 15c: corner portion
13d, 15d: lead-out part
14: power supply part
15: second electrode
16: shielding part
16a, 56a: tip portion
16b: rear end part
18: flow passage
20: first electrode receiving member
20a, 24a: front surface
20b: back surface
22: first flow passage plate
22a, 23a: opening portion
23: second flow passage plate
24: second electrode receiving member
25: inflow port
26: outflow port
50: calculation model
52: electrode pair element
52a: upstream side
52b: downstream side
53: flow passage element
54: first electrode element
54b: center
54c: end part
54d, 55d: surface
55: second electrode element
56: shielding part element
D: distance between electrodes
D₁, D₂, DL₁, DL₂: distance
DL: distance
Df: direction
L: electrode length
LN, Lm: end part plane
Lf: electrode length
Lfm: length
Mm: total length
Q: suspension
dm, tm: thickness
W: width
Wf: electrode width
X: second direction
Xm: longitudinal direction
Y: first direction
Ym: direction
γ: spacing
δ, δm: distance

## Claims

1. An electroporation device which introduces a bioactive substance into a biologically derived material in a suspension containing the biologically derived material and the bioactive substance, the electroporation device comprising:
an electrode pair in which electrodes having electrode surfaces are arranged with the electrode surfaces facing each other;
a power supply part which applies a voltage to the electrode pair; and
a shielding part which has a higher volume resistivity than a volume resistivity of the suspension,
wherein at least a part of the shielding part is disposed in a flow passage between the facing electrode surfaces, and is disposed at a region where a distance from end part planes which are obtained by connecting outer edges of the facing electrode surfaces in a first direction orthogonal to both the facing electrode surfaces is within 2 mm in a second direction orthogonal to the first direction and parallel to the electrode surfaces.

2. The electroporation device according to claim 1, further comprising:
a container for accommodating the suspension,
wherein the electrodes having the electrode surfaces are disposed in the container with the electrode surfaces facing each other,
the at least a part of the shielding part is disposed in the flow passage between the facing electrode surfaces which are disposed in the container, and is disposed at the region where the distance from the end part planes is within 2 mm in the second direction, and
a voltage is applied to the facing electrodes by the power supply part in a state in which the suspension is accommodated in the container.

3. The electroporation device according to claim 1,
wherein the shielding part is provided on at least one of an upstream side or a downstream side of the flow passage.

4. The electroporation device according to claim 1,
wherein a plurality of the shielding parts are provided on at least one of an upstream side or a downstream side of the flow passage.

5. The electroporation device according to claim 1,
wherein, with respect to a longer distance between distances of each of the facing electrode surfaces and the shielding part, a length in the second direction from the end part plane of the shielding part to a tip portion which enters a spacing between the electrode surfaces is 1/16 or more.

6. The electroporation device according to claim 1,
wherein, with respect to a longer distance between distances of each of the facing electrode surfaces and the shielding part, a length in the second direction from the end part plane of the shielding part to a rear end part on a side opposite to the electrode surfaces is 1/2 or more.

7. The electroporation device according to claim 4,
wherein, in the plurality of the shielding parts, with respect to a longer distance between distances of each of the facing electrode surfaces and the shielding part and a distance between adjacent shielding parts in the first direction, a length in the second direction from the end part plane of the shielding part to a tip portion which enters a spacing between the electrode surfaces is 1/16 or more.

8. The electroporation device according to claim 4,
wherein, in the plurality of the shielding parts, with respect to a longer distance between distances of each of the facing electrode surfaces and the shielding part and a distance between adjacent shielding parts in the first direction, a length in the second direction from the end part plane of the shielding part to a rear end part on a side opposite to the electrode surfaces is 1/2 or more.

9. The electroporation device according to any one of claims 1 to 8,
wherein the shielding part is provided at at least two locations.

10. The electroporation device according to any one of claims 3 to 8,
wherein a width of the shielding part in a direction which is orthogonal to a direction from the upstream side to the downstream side of the flow passage and is parallel to the electrode surfaces is 90% or more and 100% or less with respect to a width of the flow passage.

11. The electroporation device according to any one of claims 1 to 8,
wherein the shielding part is in a sheet shape.

12. The electroporation device according to claim 11,
wherein the shielding part is disposed in parallel to the electrode surfaces.

13. The electroporation device according to any one of claims 1 to 8,
wherein the electrode pair is disposed with the electrode surfaces in parallel to each other, and
the shielding part is disposed at a position in which, in the facing electrode surfaces, a ratio between a distance from one electrode surface and a distance from the other electrode surface facing the one electrode surface is 3:7 to 7:3 in the first direction.

14. The electroporation device according to any one of claims 1 to 8,
wherein the electrode pair is disposed with the facing electrode surfaces in parallel to each other, and
the outer edges of the electrode surfaces match each other in the second direction.

15. The electroporation device according to any one of claims 1 to 8,
wherein the electrode pair is disposed with the electrode surfaces in parallel to each other, and
the shielding part is disposed at a position of 0.01 mm or more and 3 mm or less from one electrode surface in the first direction.

16. The electroporation device according to any one of claims 1 to 8,
wherein a thickness of the shielding part is 0.5 mm or less.

17. The electroporation device according to any one of claims 1 to 8,
wherein an elastic modulus of the shielding part is 0.1 GPa or more.

18. The electroporation device according to any one of claims 1 to 8,
wherein the volume resistivity of the shielding part is 1 × 10¹⁰ Ω·cm or more.

19. An electroporation method comprising:
performing electroporation using the electroporation device according to any one of claims 1 to 8.
